# EUROPEAN PATENT APPLICATION

(11) **EP 2 741 073 A2**
(43) Date of publication of application: **11.06.2014**
(21) Application number: 13195975.1
(22) Date of filing: 06.12.2013
(51) Int. Cl.: G01N 21/64

(54) **Optical imaging apparatus**

(30) Priority: 07.12.2012 JP 2012268502
(71) Applicant: Shimadzu Corporation, Kyoto-shi Kyoto 604-8511 (JP); Tokyo University of Science Foundation, Tokyo 162-8601 (JP)
(72) Inventor: Sasayama, Tomoki, Kyoto-shi Kyoto 604-8511 (JP); Yamamoto, Satoshi, Kyoto-shi Kyoto 604-8511 (JP); Soga, Kohei, Tokyo Tokyo 162-8601 (JP)
(74) Representative: Kilian Kilian & Partner

(57) **Abstract**

Measuring light emitted from a laser light source 1 and split into four light rays through optical fibers is emitted into a space from emission units 3, and bent upward at a mirror 4. The measuring light passes through a window in the center of the sample stage 5 and strikes a lower surface of the biological sample 7 placed on the sample stage 5. A portion of fluorescence emitted through excitation by the measuring light passes through the window, is bent in an opposite direction from the measuring light by the mirror 4, and guided to a fluorescence camera 12. An objective lens 10, and a spectroscopic unit 11 for separating visible wavelength components are horizontally arranged between the camera 12 and the mirror 4. Although the projection of the measuring light and the detection of the fluorescence are performed perpendicularly to the biological sample 7, the optical components and elements are horizontally arranged, whereby the height and weight of the apparatus are reduced.

## Description

### TECHNICAL FIELD

The present invention relates to an optical imaging apparatus which projects light onto a biological sample, and two-dimensionally detects various types of light such as transmitted light, reflected light, and fluorescence obtained from the sample in response to the projected light to thereby acquire an image showing the optical properties or characteristics of the sample.

### BACKGROUND ART

When excitation light emitted from a light source (such as a halogen lamp or a semiconductor laser) is projected onto a small animal (such as a mouse or a rat) to which a fluorescent-labeled probe has been administered, fluorescence is emitted from the portion where the fluorescent-labeled probe is accumulated, such as an organ. The fluorescence is detected by a highly-sensitive camera, and an image of its intensity distribution is created. Accordingly, a distribution or a behavior of a target molecular species in the small animal in vivo can be non-invasively investigated. Various apparatuses for performing such a measurement have been known, such as the optical imaging apparatuses described in Patent Document 1 as well as in Non-Patent Documents 1 and 2.

There are two typical configurations used in conventional optical imaging apparatuses. In one configuration, a biological sample (e.g. a small animal) placed on a substantially horizontal upper surface of a stage is illuminated with light from above, and reflected light thereof or fluorescence emitted through excitation by the illuminating light is detected by a camera located above the biological sample. In the other configuration, the biological sample placed on the stage is illuminated with light from below, and fluorescence emitted through excitation by the illuminating light is detected by the camera that is arranged above the biological sample. To avoid the influence of external light, the optical system including the optical path from the light source to the camera is entirely accommodated in a housing having high light-blocking properties.

However, in the conventional optical imaging apparatus employing the optical system having the previously described configuration, various optical components for detecting the light from the sample, such as a collecting lens system, an optical filter, and a camera, are arranged along a vertically extending axis. Thus, the apparatus tends to have a larger height. In general, when only the height of the apparatus is increased, the apparatus becomes unstable and may fall down. It is thus necessary to increase the width and/or depth of the apparatus according to the height. Therefore, the conventional optical imaging apparatus is so large and heavy that a large installation area needs to be ensured, and the floor of the installation area needs to have an adequately high weight resistance. Such an apparatus is also inconvenient for use in many different locations.

### BACKGROUND ART DOCUMENT

### PATENT DOCUMENT

[Patent Document 1] JP-A 2009-257777

### NON-PATENT DOCUMENT

[Non-Patent Document 1] Yajima et al., "Development of "Clairvivo OPT" in-vivo fluorescence imaging system for small animals", Shimadzu Hyouron (Shimadzu Review), Shimadzu Hyouron Henshuu-bu, September 30, 2009, vol. 66, Nos. 1 and 2, pp. 21-27
[Non-Patent Document 2] "IVIS Imaging System (Caliper Life Sciences, Inc.)", Summit Pharmaceuticals International Corporation, [online], [searched on November 22, 2012], Internet <URL: http://www.summitpharma.co.jp/japanese/service/products/xenogen/index.html>

### DISCLOSURE OF THE INVENTION

### PROBLEMS TO BE SOLVED BY THE INVENTION

The present invention has been made to solve the previously described problems, and a main objective thereof is to provide a small, light-weight, and easily-handled optical imaging apparatus by devising the configuration of its optical system.

### MEANS FOR SOLVING THE PROBLEMS

To attain the previously described objective, the present invention provides an optical imaging apparatus which projects light onto a biological sample, and detects light obtained from the sample in response to the projected light to create a two-dimensional image, the apparatus including:
a) a sample stage on which a biological sample is to be placed in a substantially horizontal position;
b) an illuminating unit that emits measuring light to be projected onto the biological sample on the sample stage;
c) an imaging unit that acquires an image of at least either reflected light or fluorescence emitted from the biological sample in response to the measuring light from the illuminating unit; and
d) a light-guiding optical system including a common reflection optical unit that bends both the measuring light and the emitted light within a space so as to guide the measuring light from the illuminating unit to the biological sample, and guide the emitted light from the biological sample to the imaging unit.

In the optical imaging apparatus according to the present invention, the measuring light emitted from the illuminating unit strikes the reflection optical unit of the light guiding optical system. The optical path is thereby bent so that the measuring light is projected onto the biological sample placed on the sample stage. The reflected light from the biological sample in response to the measuring light, or a portion of the fluorescence emitted from the sample through excitation by the measuring light travels in an opposite direction from the measuring light to strike the reflection optical unit. The optical path is thereby bent so that the reflected light or the portion of the fluorescence reaches the imaging unit. The common reflection optical unit bends both the measuring light and the emitted light in the previously described manner. Thus, for example, even in the case where the measuring light is projected onto the biological sample from above or below the sample, it is not necessary to vertically arrange optical components and elements included in the illuminating unit or optical components and elements included in the imaging unit. The optical components and elements may be arranged, for example, in the horizontal direction. Accordingly, the height of the apparatus can be suppressed.

In a preferable mode of the optical imaging apparatus according to the present invention, the illuminating unit includes a plurality of emission units that are arranged so as to surround an optical axis of the emitted light directed toward the imaging unit from the reflection optical unit, and the measuring light is projected onto the biological sample from the reflection optical unit along a path substantially coaxial with the optical axis of the emitted light directed toward the reflection optical unit from the biological sample. More preferably, the plurality of emission units may be arranged at substantially equal intervals of rotation angle so as to surround the optical axis of the emitted light directed toward the imaging unit from the reflection optical unit.

In this configuration, even if the illuminating unit is not located on the optical axis of the emitted light directed toward the imaging unit from the reflection optical unit, the measuring light can be projected onto the biological sample placed on the sample stage with a substantially uniform intensity. Accordingly, for example, when a fluorescence intensity distribution image derived from a fluorescent material that has been administered to the biological sample is acquired by using the measuring light as excitation light, an accurate fluorescence intensity distribution image which is free from dependency on the excitation light intensity distribution can be obtained.

Each of the plurality of emission units may include a light source, or alternatively, the measuring light emitted from one light source (such as a high-brightness semiconductor laser) may preferably be split into a plurality of light rays by optical fibers or the like, so as to be transmitted to the respective emission units and emitted into the space from the emission units. Such a system is advantageous in that the same wavelength of the measuring light is emitted from all the respective emission units and that the production cost of the apparatus can be suppressed. The respective emission units may preferably generate a spatially-diverging laser light beam by using an optical refractive element such as a lens array because of the reason which will be described later.

In one preferable mode of the optical imaging apparatus according to the present invention,
the sample stage includes a light transmitting portion at least in a section of an area where the biological sample is placed, and
at least the reflection optical unit is arranged below the sample stage, the measuring light bent at the reflection optical unit is projected onto a lower surface of the biological sample through the light transmitting portion, and the emitted light from the biological sample reaches the reflection optical unit through the light transmitting portion and is bent at the reflection optical unit to be guided to the imaging unit.

In this configuration, since the reflection optical unit is not arranged in the space above the sample stage, the structure above the sample stage can easily be constructed in an openable form. Employing the structure allows the biological sample to be placed on the sample stage from above. It also acquisition of a fluorescence image and/or a visible light image of the abdominal side of a biological sample being held in a natural body position.

The optical imaging apparatus according to the present invention may further include an optical splitting unit that splits the emitted light from the biological sample into light rays in a plurality of wavelength ranges, wherein the imaging unit may include a plurality of imaging elements that respectively acquire images formed by the light rays in a plurality of wavelength ranges split by the optical splitting unit.

For example, when the emitted light from the biological sample includes fluorescence in an infrared or near-infrared wavelength range and reflected light in a visible wavelength range, the apparatus having the previously described configuration can simultaneously obtain a fluorescence image and a visible light image of the biological sample. Since the emitted light also includes reflected light of the excitation light, an excitation light wavelength range and a fluorescence wavelength range may be separated to take an image in each of the wavelength ranges. In this case, a two-dimensional intensity distribution of the excitation light is obtained, which can be used to evaluate the uniformity of the excitation light projection intensity, or to perform an adjustment for making the excitation light projection intensity uniform.

In the optical imaging apparatus according to the present invention, the illuminating unit may employ an optical refractive element for producing a spatially diverged beam of laser light. For example, the optical refractive element may include a lens array. In general, the laser light beam emitted from a light source such as a semiconductor laser has a high energy density, but its spot size is small. By diverging the laser light beam into a broader beam of light using the optical refractive element such as the lens array, a relatively large range can be illuminated with a substantially uniform light intensity.

The optical imaging apparatus according to the present invention may further include:
a moving mechanism unit that produces a relative motion between the sample stage and a light measurement system including the illuminating unit, the light guiding optical system, and the imaging unit, in one-dimensional or two-dimensional directions parallel with a placement surface of the sample stage; and
an operation unit to be operated by a measurer to control the moving mechanism unit so that the light measurement system and the sample stage are brought into a desired positional relationship,
wherein a two-dimensional image of light obtained from any portion of the biological sample placed on the sample stage can be acquired by the measurer performing an appropriate operation on the operation unit.

Here, the moving mechanism unit may not include a driving source such as a motor, and the sample stage or the light measurement system may be manually and mechanically moved according to the amount of operation or the like on the operation unit by the measurer. However, it is more preferable to automatically move the sample stage or the light measurement system by an electrical control.

Thus, one preferable mode of the optical imaging apparatus according to the present invention further includes a driving source included in the moving mechanism unit and a control unit that controls driving of the driving source according to the operation on the operation unit by the measurer.

In accordance with these configurations, even when the sample is much larger than a range that can be imaged by the light measurement system, the measurer can appropriately select a portion to be observed or measured by operating the operation unit. Of course, a fluorescence image or the like covering a wider range can be obtained by repeating the observation or measurement while operating the operation unit.

The optical imaging apparatus according to the present invention may further include:
a moving mechanism unit that produces a relative motion between the sample stage and a light measurement system including the illuminating unit, the light guiding optical system, and the imaging unit in one-dimensional or two-dimensional directions parallel with a placement surface of the sample stage; and
an imaging control unit that acquires an image formed by the emitted light from the biological sample for a plurality of times in the imaging unit while one-dimensionally or two-dimensionally moving at least either the light measurement system or the sample stage by using the moving mechanism unit.

In accordance with the configuration, even when the sample is much larger than a range that can be imaged by the light measurement system, the fluorescence image or the like can be automatically acquired over a larger range than the range that can be imaged, without requiring the measurer to perform a troublesome operation or task.

Especially, the previously described configuration may further include an image forming unit that reproduces a two-dimensional image of a range larger than a range obtained by one cycle of imaging operation by the imaging unit by coupling a plurality of images acquired under control of the imaging control unit. This system can provide one image obtained by piecing together a plurality of images showing different portions rather than displaying those images individually.

### EFFECTS OF THE INVENTION

In accordance with the optical imaging apparatus according to the present invention, both the measuring light projected onto the biological sample and the emitted light obtained from the biological sample are bent by the common reflection optical unit. Thus, even in a configuration in which the measuring light is projected onto the biological sample from immediately above or below the biological sample, that is, along an axis extending in a vertical direction, and the emitted light is obtained from the biological sample along the same axis, various optical components and elements included in the illuminating unit or the imaging unit can be arranged not in the vertical direction, but in a horizontal direction. Accordingly, the height of the apparatus can be suppressed, and the entire apparatus can be made compact. Suppressing the height of the apparatus also improves its stability and prevents an unnecessary increase in its weight. Thus, a weight reduction of the apparatus can also be achieved.

The optical imaging apparatus according to the present invention can particularly be configured so that the measuring light is projected from below and the emitted light directed downward is observed. In this case, when the biological sample is a small animal such as a mouse or a rat, its abdominal side can be observed in a natural body position with its abdomen oriented downward, not facing upward in an unnatural body position. Accordingly, an excessive burden on the small animal can be avoided, which improves the reliability of the measurement.

### BRIEF DESCRIPTION OF THE DRAWINGS

Fig. 1 is an external perspective view of an optical imaging apparatus according to one embodiment of the present invention, with a sample mounting upper lid being opened.
Fig. 2 is a schematic side view of the optical imaging apparatus according to the present embodiment, with an outer housing removed.
Fig. 3 is a configuration diagram of an optical system in the optical imaging apparatus according to the present embodiment.
Fig. 4 is a view illustrating the arrangement of emission units and visible light emitting units in the optical imaging apparatus according to the present embodiment.
Fig. 5 is a configuration diagram of a main portion of an optical imaging apparatus according to another embodiment of the present invention.
Fig. 6 is a configuration diagram of a main portion of an optical imaging apparatus according to yet another embodiment of the present invention.

### MODES FOR CARRYING OUT THE INVENTION

Hereinafter, an optical imaging apparatus according to one embodiment of the present invention is described with reference to the attached drawings.

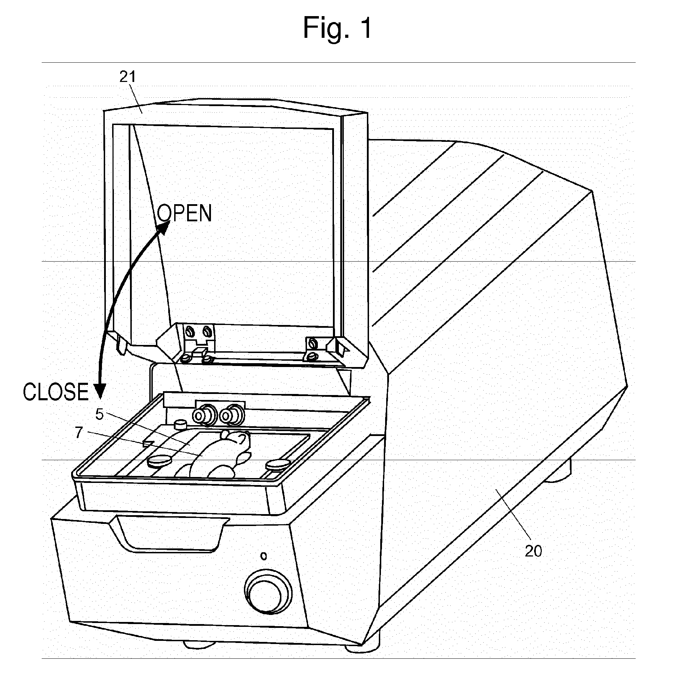

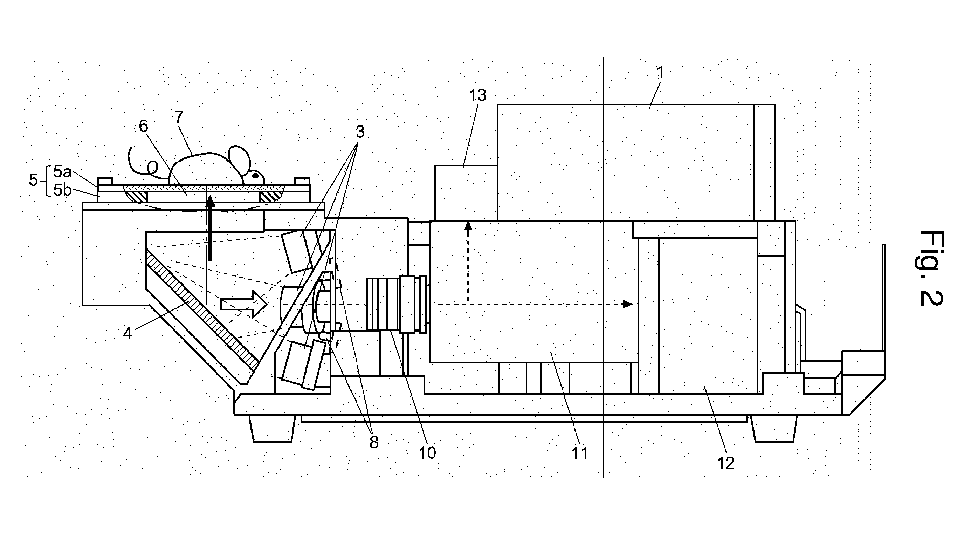

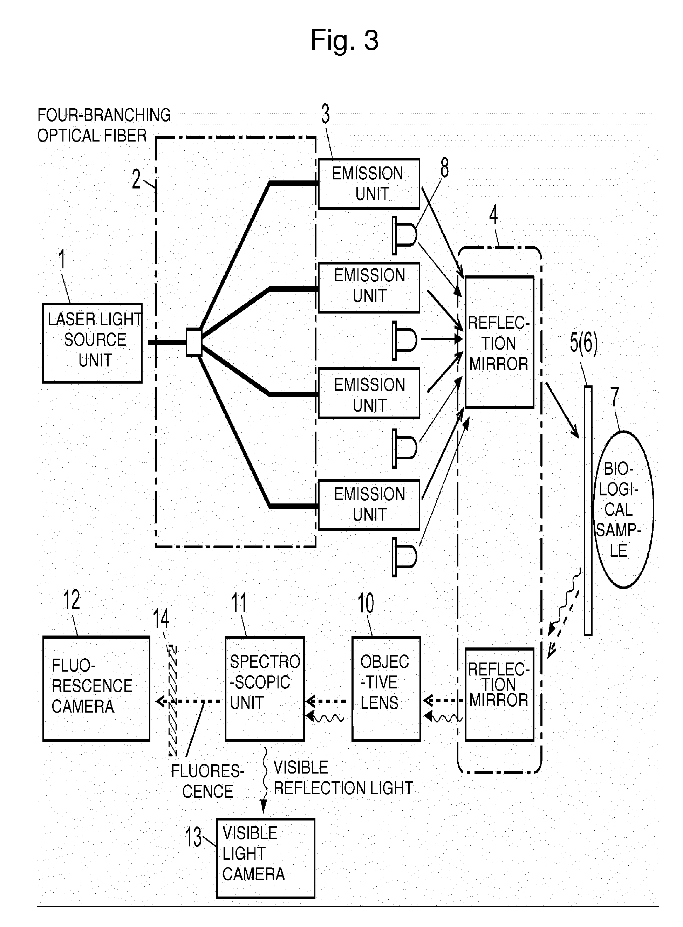

Fig. 1 is an external perspective view of an optical imaging apparatus according to one embodiment of the present invention, with a sample mounting upper lid being opened. Fig. 2 is a partially-broken schematic side view of the optical imaging apparatus according to the present embodiment, with an outer housing removed. Fig. 3 is a configuration diagram of an optical system in the optical imaging apparatus according to the present embodiment. That is, Fig. 2 illustrates the spatial arrangement of the components and elements of the optical system in the optical imaging apparatus according to the present embodiment, and Fig. 3 illustrates the functional configuration of the optical system.

As shown in Fig. 1, the optical imaging apparatus according to the present embodiment has a substantially box-like shape longer in the depth direction than in the width and height directions. A sample mounting upper lid 21 capable of moving upward on a hinge is provided in a front portion of the upper surface of an outer housing 20. When the sample mounting upper lid 21 is opened, a sample stage 5 whose upper surface is substantially horizontal is exposed. A biological sample 7 as the subject of the measurement, such as a mouse or a rat, can be placed on the sample stage 5. When the sample mounting upper lid 21 is opened, there is no obstacle above the sample stage 5. Thus, a measurer can very easily place or remove the biological sample 7.

As shown in Fig. 2, the sample stage 5 includes a metal support 5a where a large rectangular opening window 6 is formed in a center portion, and a transparent plate 5b, such as a glass plate , that is attached onto the support 5a. The transparent plate 5b and the window 6 allow a lower surface of the biological sample 7, i.e., an abdominal surface of the biological sample 7 in a normal placement state, to be seen through from below. In a space below the sample stage 5, a reflection mirror 4 corresponding to a reflection optical unit in the present invention, and four emission units 3 respectively corresponding to emission units in the present invention are arranged. An objective lens 10, a spectroscopic unit 11, a fluorescence camera 12 and other components are also arranged behind the space (the right side in Fig. 2) where the previously mentioned components are arranged. A laser light source unit 1, and a visible light camera 13 are arranged above the components 10, 11, and 12. Some optical components and elements (which will be described later) are not shown in Fig. 2.

The configuration of the optical system in the optical imaging apparatus according to the present embodiment, and the functions of the respective optical components and elements are hereinafter described with reference to Fig. 3 in addition to Fig. 2.

Before measurement, a probe labeled with a predetermined fluorescent material is administered to the biological sample 7, such as a mouse, that is the subject of measurement. A measurer opens the sample mounting upper lid 21 as shown in Fig. 1, places the biological sample 7 in vivo on the sample stage 5, and closes the sample mounting upper lid 21.

When the laser light source unit 1 is energized according to an instruction to start measurement from the measurer, measuring light (in this case, excitation light) emitted from the laser light source unit 1 is split into four light rays through an optical fiber 2, and then guided to the four emission units 3. Each of the emission units 3 includes an optical refractive element that is a lens array, and emits diverging light so as to spread, over a predetermined angle, the light with a relatively small diameter from the terminal end of the optical fiber 2, and to reduce intensity unevenness in an illumination region. The four emission units 3 are arranged substantially symmetrical with respect to the optical axis of the objective lens (which will be described later) at an interval of a rotation angle of 90°.

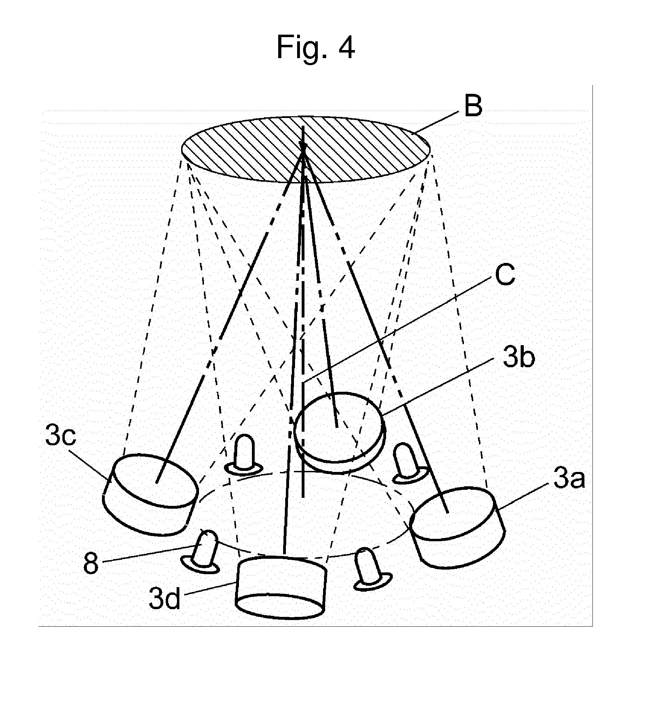

The schematic arrangement of the four emission units 3 (3a, 3b, 3c and 3d) and visible light emitting units 8 is shown in Fig. 4. The four emission units 3 (3a, 3b, 3c and 3d) are fixed obliquely at a predetermined angle such that the optical axis of each light emitted therefrom is directed inward, that is, approaching the optical axis C. Accordingly, the rays of light emitted from the four emission units 3 overlap each other at a position a predetermined distance apart from the four emission units 3 to form an illumination region B with a substantially uniform light intensity.

As shown in Fig. 2, the reflection mirror 4 that is fixed obliquely at an angle of 45° with respect to the optical axis C is arranged on the front side of a traveling direction of the measuring light emitted into the space from the four emission units 3. Therefore, each measuring light strikes the reflection mirror 4 to be bent upward at a substantially right angle. The measuring light is transmitted through the window 6 and the transparent plate 5b of the sample stage 5 to strike the lower surface of the biological sample 7. Since the biological sample 7 is normally placed as shown in Figs. 1 and 2, the measuring light strikes the abdominal surface of the biological sample 7.

The rays of measuring light emitted from the four emission units 3 (3a, 3b, 3c and 3d) overlap each other on a plane near the upper surface of the sample stage 5. Thus, the biological sample 7 is illuminated with a substantially uniform light intensity. Upon illumination with this light, the fluorescent material in the biological sample 7 is excited to emit fluorescence. The fluorescence is emitted not only in one direction but in various directions. A portion of the fluorescence emitted downward is transmitted through the transparent plate 5b and the window 6, and strikes the reflection mirror 4 located below. The fluorescence is bent at a substantially right angle by the reflection mirror 4 in an opposite direction from the measuring light (the excitation light) and guided to the objective lens 10. The fluorescence focused by the objective lens 10 is introduced into the fluorescence camera 12 through the spectroscopic unit 11, where the fluorescence forms an image on an image sensor of the camera 12. Accordingly, in the fluorescence camera 12, a two-dimensional image showing an intensity distribution of the fluorescence from the biological sample 7 is created. The image is displayed on a monitor (not shown) and recorded.

The light reaching the objective lens 10 includes not only a wavelength component of the fluorescence derived from the biological sample 7, but also that of the excitation light. In the case where the image sensor installed in the fluorescence camera 12 has no sensitivity to the wavelength component of the excitation light, no problem occurs even when the excitation light enters the fluorescence camera 12. However, in the case where the image sensor has sensitivity to the wavelength component of the excitation light, it is necessary to remove the wavelength component of the excitation light before the light enters the camera 12. Thus, in this case, an optical filter 14 having such wavelength characteristics as to remove the wavelength component of the excitation light may be arranged on the optical path before the light enters the fluorescence camera 12 (between the spectroscopic unit 11 and the fluorescence camera 12 in the example of Fig. 3) so as to remove the unnecessary wavelength component of the excitation light by the optical filter 14.

The visible light emitting units 8 such as visible light LEDs respectively arranged between the four emission units 3 emit visible light in a predetermined wavelength range in substantially the same direction as the measuring light. Similarly to the previously described measuring light, the visible illuminating light emitted from the visible light emitting units 8 is bent upward by the reflection mirror 4, and strikes the lower surface of the biological sample 7. Reflected light for the visible illuminating light travels downward from the biological sample 7, is bent in a substantially traverse direction by the reflection mirror 4, and guided to the objective lens 10 in a similar manner to the aforementioned portion of the fluorescence. After passing through the objective lens 10, the visible reflected light is introduced into the spectroscopic unit 11 in a similar manner to the fluorescence. The spectroscopic unit 11 includes a two-color mirror that selectively reflects only the wavelength components of the visible light while allowing transmission of near-infrared to infrared wavelength components. Therefore, the introduced visible reflected light is bent upward by the dichroic mirror, and guided to the visible light camera 13. Accordingly, a visible light image of the biological sample 7 is obtained in the visible light camera 13.

As described thus far, the optical imaging apparatus according to the present embodiment can acquire the visible light image and the fluorescence image of the lower surface, i.e., the abdominal side of the biological sample 7 at the same time. Of course, it is not essential to acquire the visible light image so as to perform fluorescence imaging.

In the optical imaging apparatus according to the present embodiment, the common reflection mirror 4 is used to bend each of the four rays of light at substantially 90°: the measuring light and the visible illuminating light projected onto the biological sample 7, as well as the fluorescence and the reflected light obtained from the sample 7. This design has made it possible to horizontally arrange the optical components and elements of the illumination optical system (such as the emission units 3) and those of the detection optical system (such as the objective lens 10, the spectroscopic unit 11, and the fluorescence camera 12) while performing both the projection of the measuring light onto the biological sample 7 and acquisition of the fluorescence from the sample 7 in the vertical direction. Consequently, the height of the apparatus can be suppressed as compared to a conventional apparatus, and the apparatus can be reduced in size and weight.

Another advantage exists in that the abdominal side of a small animal in a natural body position can be observed since both the projection of the measuring light or the visible illuminating light onto the lower surface of the biological sample 7 placed on the sample stage 5 and the detection of the fluorescence or the reflected light emitted from the lower surface are performed in the space below the sample.

In the configuration of the previous embodiment, the reflection mirror 4 is a plane mirror. By giving power to the reflection mirror 4, i.e. by using a concave mirror or a convex mirror, the imaging operation can be performed with the size of a measurement field or an illumination field (i.e., an illumination range or an imaging range) adjusted. When a plane mirror, a concave mirror and a convex mirror are provided so that they can be switched from or replaced with one another as the reflection mirror 4, a more appropriate image can be acquired according to the purpose of imaging.

In the previous embodiment, a fluorescence image and a visible light image of the biological sample 7 can be acquired. A two-dimensional intensity distribution of the excitation light can also be obtained by extracting the wavelength component of the excitation light in the spectroscopic unit 11, introducing the excitation light into yet another camera, and creating an image thereof,. For example, the two-dimensional intensity distribution of the excitation light can be used for allowing a measurer to visually check whether there is illumination unevenness of the excitation light, or for automatically adjusting the projection so as to reduce the illumination unevenness of the excitation light.

Some fluorescent materials emit fluorescence at a plurality of different wavelengths in response to one excitation light, and such a fluorescent material can also be used for the labelling. In this case, the plurality of wavelengths of fluorescence can be separately extracted and respectively introduced into separate cameras to form an image in each camera. Accordingly, a two-dimensional distribution image can be obtained for each of the different wavelengths of fluorescence.

Furthermore, the optical system including the reflection mirror 4, which is arranged below the sample stage 5 in the previous embodiment, may be collectively arranged above the sample stage 5.

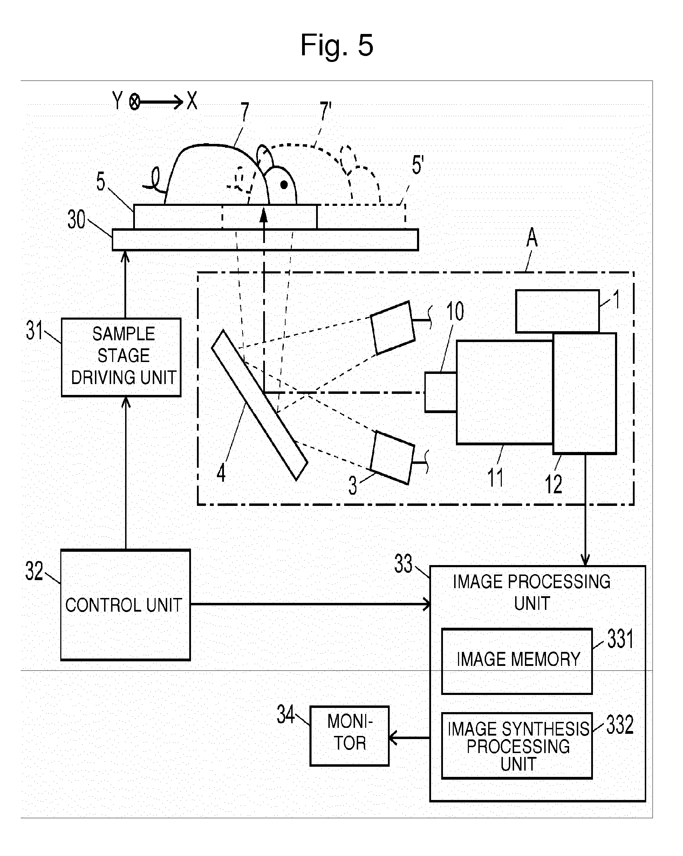

Next, an optical imaging apparatus according to another embodiment of the present invention is described with reference to Fig. 5. Fig. 5 is a configuration diagram of a main portion of the optical imaging apparatus according to the present embodiment. In the optical imaging apparatus according to the previous embodiment, the fluorescence intensity distribution image is obtained in a predetermined range of the biological sample 7 that is placed on the sample stage 5, and the imaging range is predetermined. Thus, for example, if the biological sample has a large size, and a range to be imaged in the sample is larger than the imaging range which is predetermined in the apparatus, it is necessary to perform the imaging operation a plurality of times while changing the position of the biological sample on the sample stage. The optical imaging apparatus according to the present embodiment is designed to reduce the time and labor for such an operation.

In the optical imaging apparatus according to the present embodiment, the sample stage 5 can be moved in the directions of the two axes of X and Y which are parallel to the placement surface on the sample stage 5 and perpendicular to each other by a sample stage moving mechanism 30 including a motor, slide rails and other elements. A sample stage driving unit 31 activates the motor in the sample stage moving mechanism 30 in response to a control signal from a control unit 32 to thereby move the sample stage 5 to any position on the X and Y axes (e.g., a position denoted by reference numeral 5' in Fig. 5). A light measurement system A including the laser light source unit 1, the emission units 3, the reflection mirror 4, the objective lens 10, the spectroscopic unit 11, and the fluorescence camera 12 is fixed in position. Thus, when the sample stage 5 is moved as described previously, the projection range of the excitation light on the lower surface of the biological sample 7 that is placed on the sample stage 5 (or 5') is changed. The imaging range of the emitted excitation light intensity is also thereby changed.

In the optical imaging apparatus according to the present embodiment, a fluorescence intensity distribution image over a wide range of the biological sample 7 can be obtained as follows.

That is, the control unit 32 controls the sample stage driving unit 31 so that the sample stage 5 comes to a predetermined initial position. The sample stage driving unit 31 thereby sends a driving signal to the motor in the sample stage moving mechanism 30. As a result, the sample stage 5 is brought to the initial position. In this state, the fluorescence camera 12 takes a fluorescence intensity distribution image based on the fluorescence coming from the biological sample 7, and sends obtained image data to an image processing unit 33. The image processing unit 33 temporarily stores the received image data in an image memory 331.

When one cycle of imaging operation is completed, the control unit 32 controls the sample stage driving unit 31 so that the sample stage 5 is moved by a predetermined distance along the X axis and/or the Y axis. After the sample stage 5 is moved, the fluorescence camera 12 once more acquires a fluorescence intensity distribution image. By repeating the aforementioned operation a plurality of times over a predetermined range, an image covering a predetermined range larger than one imaging range can be obtained with no omission. When the entire imaging operation is completed, an image synthesis processing unit 332 in the image processing unit 33 reads out image data for different imaging ranges from the image memory 331, pieces together all the images by, for example, determining the portions partially overlapped with each other in those images, and creates a fluorescence intensity distribution image corresponding to the predetermined range. The image is displayed on a monitor 34. Of course, the obtained images may be individually presented on the monitor 34 without being pieced together in the previously described manner.

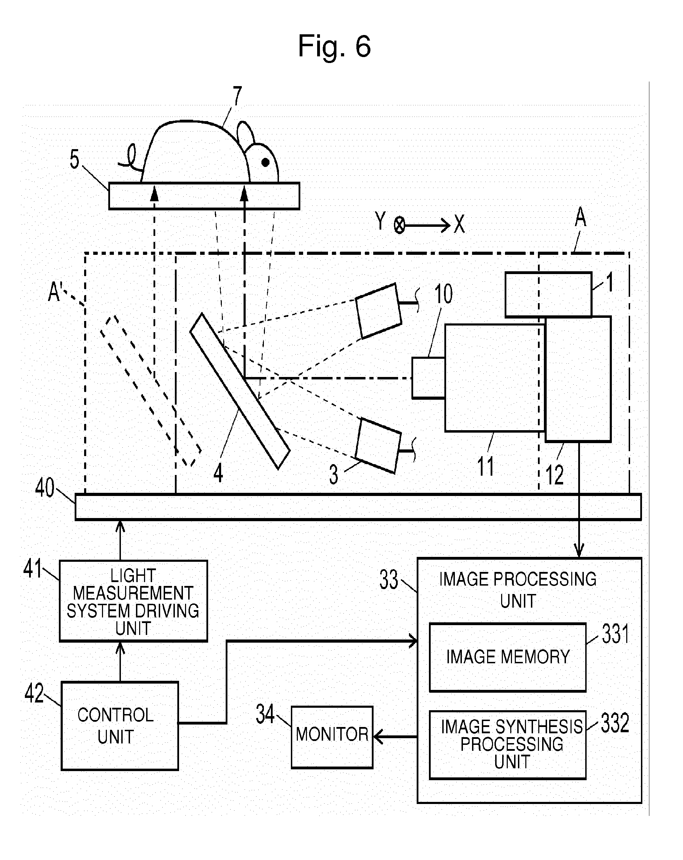

The configuration of a main portion of an optical imaging apparatus according to another embodiment which can perform a similar imaging operation is shown in Fig. 6. In the embodiment shown in Fig. 6, the position of the sample stage 5 is fixed, and the entire light measurement system A can be moved in the directions of the two axes of X and Y by a light measurement system moving mechanism 40. A light measurement system driving unit 41 is controlled by a control unit 42 to activate a motor in the light measurement system moving mechanism 40. The light measurement system A is thereby moved to any position on the X axis and the Y axis (e.g., a position denoted by reference character A' in Fig. 6). It is obvious that the position of the imaging range on the biological sample 7 that is placed on the sample stage 5 can also be changed by the configuration, and an imaging operation similar to that described in the embodiment shown in Fig. 5 can be performed.

Both of the embodiments shown in Figs. 5 and 6 are designed to automatically take an image which covers a larger range than the imaging range by the fluorescence camera12. It is also possible to create an apparatus which does not automatically set the imaging range but according to an operation by a measurer. For example, in the configuration in Fig. 5, when a measurer operates an operation unit provided in the control unit 32, the control unit 32 sends a control signal to the sample stage driving unit 31 according to the amount of operation or the like. The sample stage driving unit 31 activates the motor in the sample stage moving mechanism 30 according to the control signal to thereby move the sample stage 5 by a predetermined distance along the X axis and/or the Y axis. When the sample stage 5 is moved relative to the light measurement system A, the position of the imaging range changes. As a result, the position of the fluorescence intensity distribution image displayed on the monitor 34 changes. The measurer can observe a desired portion of the biological sample 7 while viewing the displayed image.

An even simpler configuration is such that the sample stage moving mechanism 30 or the light measurement system moving mechanism 40 does not have any driving source such as a motor, and a measurer turns or slides a knob to move the sample stage 5 or the light measurement system A by a manual driving force.

Although the sample stage 5 or the light measurement system A is moved in two dimensions in the embodiments in Figs. 5 and 6, it is possible to adopt a mechanism which moves the sample stage 5 or the light measurement system A along a single direction.

The previously described embodiments are merely examples of the present invention. Obviously, various modifications, changes and additions appropriately made within the spirit of the present invention are included within the scope of claims of the present patent application, in addition to the previously described variations.

### EXPLANATION OF NUMERALS

- 1: Laser Light Source Unit
- 2: Optical Fiber
- 3, 3a, 3b, 3c, 3d: Emission Unit
- 4: Reflection Mirror
- 5: Sample Stage
- 5a: Support
- 5b: Transparent Plate
- 6: Opening Window
- 7: Biological Sample
- 8: Visible Light Emission Unit
- 10: Objective Lens
- 11: Spectroscopic Unit
- 12: Fluorescence Camera
- 13: Visible Light Camera
- 14: Optical Filter
- 20: Outer Housing
- 21: Sample Mounting Upper Lid
- 30: Sample Stage Moving Mechanism
- 31: Sample Stage Driving Unit
- 32, 42: Control Unit
- 33: Image Processing Unit
- 331: Image Memory
- 332: Image Synthesis Processing Unit
- 34: Monitor
- 40: Light Measurement System Moving Mechanism
- 41: Light Measurement System Driving Unit

## Claims

1. An optical imaging apparatus which projects light onto a biological sample, and detects light obtained from the sample in response to the projected light to create a two-dimensional image, the apparatus comprising:
a) a sample stage on which a biological sample is to be placed in a substantially horizontal position;
b) an illuminating unit that emits measuring light to be projected onto the biological sample on the sample stage;
c) an imaging unit that acquires an image of at least either reflected light or fluorescence emitted from the biological sample in response to the measuring light from the illuminating unit; and
d) a light-guiding optical system including a common reflection optical unit that bends both the measuring light and the emitted light within a space so as to guide the measuring light from the illuminating unit to the biological sample, and guide the emitted light from the biological sample to the imaging unit.

2. The optical imaging apparatus according to claim 1,
wherein the illuminating unit includes a plurality of emission units that are arranged so as to surround an optical axis of the emitted light directed toward the imaging unit from the reflection optical unit, and the measuring light is projected onto the biological sample from the reflection optical unit along a path substantially coaxial with the optical axis of the emitted light directed toward the reflection optical unit from the biological sample.

3. The optical imaging apparatus according to claim 1 or 2,
wherein the sample stage includes a light transmitting portion at least in a section of an area where the biological sample is placed, and
at least the reflection optical unit is arranged below the sample stage, the measuring light bent at the reflection optical unit is projected onto a lower surface of the biological sample through the light transmitting portion, and the emitted light from the biological sample reaches the reflection optical unit through the light transmitting unit and is bent at the reflection optical unit to be guided to the imaging unit.

4. The optical imaging apparatus according to any one of claims 1 to 3, further comprising
an optical splitting unit that splits the emitted light from the biological sample into light rays in a plurality of wavelength ranges, wherein the imaging unit includes a plurality of imaging elements that respectively acquire images formed by the light rays in a plurality of wavelength ranges split by the optical splitting unit.

5. The optical imaging apparatus according to any one of claims 1 to 4,
wherein the illuminating unit includes an optical refractive element for producing a spatially diverged beam of laser light beam.

6. The optical imaging apparatus according to claim 5,
wherein the optical refractive element includes a lens array.

7. The optical imaging apparatus according to any one of claims 1 to 6, further comprising:
a moving mechanism unit that produces a relative motion between the sample stage and a light measurement system including the illuminating unit, the light guiding optical system, and the imaging unit, in one-dimensional or two-dimensional directions parallel with a placement surface of the sample stage; and
an operation unit to be operated by a measurer to control the moving mechanism unit so that the light measurement system and the sample stage are brought into a desired positional relationship,
wherein a two-dimensional image of light obtained from any portion of the biological sample placed on the sample stage can be acquired by the measurer performing an appropriate operation on the operation unit.

8. The optical imaging apparatus according to claim 7, further comprising:
a driving source included in the moving mechanism unit; and
a control unit that controls driving of the driving source according to the operation on the operation unit by the measurer.

9. The optical imaging apparatus according to any one of claims 1 to 6, further comprising:
a moving mechanism unit that produces a relative motion between the sample stage and a light measurement system including the illuminating unit, the light guiding optical system, and the imaging unit in one-dimensional or two-dimensional directions parallel with a placement surface of the sample stage; and
an imaging control unit that acquires the image formed by the emitted light from the biological sample for a plurality of times in the imaging unit while one-dimensionally or two-dimensionally moving at least either the light measurement system or the sample stage by using the moving mechanism unit.

10. The optical imaging apparatus according to claim 9, further comprising
an image forming unit that reproduces a two-dimensional image of a range larger than a range obtained by one cycle of imaging operation by the imaging unit by coupling a plurality of images acquired under control of the imaging control unit.
